# EUROPÄISCHE PATENTSCHRIFT

(11) **EP 2 504 322 B1**
(45) Veröffentlichungstag und Bekanntmachung des Hinweises auf die Patenterteilung: **02.04.2014**
(21) Anmeldenummer: 09760863.2
(22) Anmeldetag: 26.11.2009
(51) Int. Cl.: C07D 261/04

(54) **VERFAHREN ZUR HERSTELLUNG 5,5-DISUBSTITUIERTER 4,5-DIHYDROISOXAZOL-3-THIOCARBOXAMIDIN-SALZE**
METHOD FOR PRODUCING 5,5-DISUBSTITUTED 4,5-DIHYDROISOXAZOL-3-THIOCARBOXAMIDINE SALTS
PROCÉDÉ DE PRODUCTION DE SELS DE 4,5-DIHYDROISOXAZOL-3-THIOCARBOXAMIDINE 5,5-DISUBSTITUÉS

(43) Veröffentlichungstag der Anmeldung: 03.10.2012
(73) Patentinhaber: BASF SE, 67056 Ludwigshafen (DE)
(72) Erfinder: FRASSETTO, Timo, 68165 Mannheim (DE)
(86) Internationale Anmeldenummer: PCT/EP2009/065925
(87) Internationale Veröffentlichungsnummer: WO 2011/063842

(56) Entgegenhaltungen:
- EP-A1- 1 767 528
- EP-A1- 1 829 868
- WO-A1-2007/096576
- MZENGEZA S ET AL: "Asymmetric Induction in Nitrone Cycloadditions: A Total Synthesis of Acivicin by Double Asymmetric Induction" JOURNAL OF ORGANIC CHEMISTRY, AMERICAN CHEMICAL SOCIETY, EASTON.; US LNKD- DOI:10.1021/JO00252A035, Bd. 53, Nr. 17, 1. Januar 1988 (1988-01-01), Seiten 4074-4081, XP002526239 ISSN: 0022-3263
- MZENGEZA, S.; YANG, C. M.; WHITNEY, R. A.: JOURNAL OF THE AMERICAN CHEMICAL SOCIETY, Bd. 109, 1987, Seiten 276-277, XP007914245

## Beschreibung

Die vorliegende, Erfindung betrifft ein Verfahren zur Herstellung 5,5-disubstituierter 4,5-Dihydroisoxazol-3-thiocarboxamidinsalze der Formel (I), 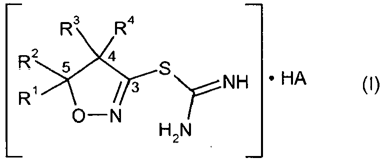 wobei R¹, R², R³, R⁴ und A folgende Bedeutungen haben:
- R¹, R²: unabhängig voneinander jeweils C₁-C₆-Alkyl oder C₁-C₄-Halogenalkyl;
- R³, R⁴: unabhängig voneinander jeweils Wasserstoff, Halogen, C₁-C₆-Alkyl oder C₁-C₄-Halogenalkyl; oder
- R¹ und R², R³ und R⁴ oder R¹ und R³: bilden zusammen eine C₂-C₅-Alkandiyl-Kette, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann; und
- A: Chlor oder Brom.

5,5-disubstituierte 4,5-Dihydroisoxazol-3-thiocarboxamidinsalze der Formel (I) sind wichtige Zwischenprodukte für die Herstellung agrochemischer und pharmazeutischer Wirkstoffe (WO2002/062770).

5,5-disubstituierte 4,5-Dihydroisoxazol-3-thiocarboxamidinsalze und Verfahren zu deren Herstellung sind aus dem Stand der Technik bekannt. EP 1 829 868 (1) beschreibt die Umsetzung 3-halogenierter 4,5-Dihydroisoxazole mit Thioharnstoff in Gegenwart von Säuren. 3-halogenierte 4,5-Dihydroisoxazole können durch 1,3-dipolare Cycloaddition von Nitriloxiden an Alkene hergestellt werden (WO2006/038657 (2), WO2000/050410 (3)). Nitriloxide werden bevorzugt aus Dihalogenformoximen in situ hergestellt und direkt weiter verarbeitet (JP2008/001597 (4)). Eine Alternative stellt die Halogenierung von 3-Isoxazolidinonen mit POCl₃ (WO2007/096576 (5)) dar.

MZENGEZA S ET AL. beschreiben in JOURNAL OF ORGANIC CHEMISTRY, AMERICAN CHEMICAL SOCIETY, EASTON.; OI:10.1021/J000252A035, Bd. 53, Nr. 17, 1. Januar 1988 (1988-01-01), Seiten 4074-4081 die Halogenierung von 5-mono-substituierten-4,5-dihydroisoxazolen in 3-Position durch Reaktion mit Chlorgas;
Eine entsprechende Umsetzung wird auch in MZENGEZA, S.; YANG, C. M.; WHITNEY, R. A. JOURNAL OF THE AMERICAN CHEMICAL SOCIETY, Bd. 109, 1987, Seiten 276-277 offenbart.

Die offenbarung von EP1767528 entspricht der von EP1829868 (Seite 1, zeile 26).

Einen Überblick gibt das folgende Schema 1, worin R und X stellvertretend für die in den Dokumenten des Standes der Technik (1), (2), (3), (4) und (5) jeweils genannten Substituenten stehen:

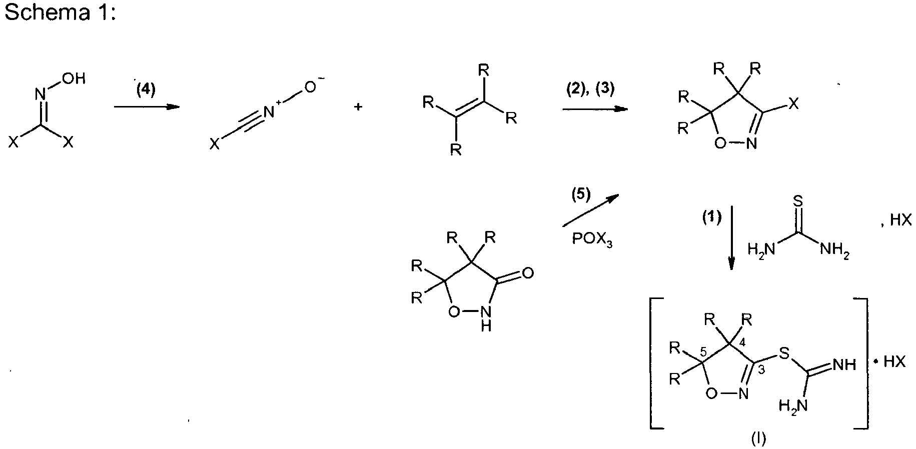

Im Rahmen der Totalsynthese von Acivicin wurde die 3-Position des 4,5-Dihydroisoxazolyl-Teils eines Aminosäureesters direkt chloriert (J. Org. Chem., 53 (17), 4074-4081).

Ein Nachteil des Verfahrens gemäß Schema 1 ist der Einsatz instabiler Dihalogenformoxime. Im Fall des Dichlorderivats zeigt sich auch nach destillativer Reinigung innerhalb weniger Tage deutliche Zersetzung (Ber. 65B, 754-759), für das Dibromderivat verläuft die Zersetzung bei Raumtemperatur langsamer, bei erhöhter Temperatur stürmisch (J. Liebigs Ann. 489, 7-30). Als Folge sind die Ausbeuten des Verfahrens häufig nur mäßig. Beide Verbindungen zerfallen darüber hinaus bei der Zersetzung zu giftigen Gasen und sind stark hautreizend. Weiterhin muss eine Destillation der Zwischenprodukte aufgrund des hohen Energieinhalts der Verbindungen unter Vorsichtsmaßnahmen erfolgen. Bei der Verwendung von 3-Isoxazolidinonen gestaltet sich die Darstellung des Ausgangsmaterials als aufwändig.
Die großtechnische Synthese der Verbindungen der Formel (I) ausgehend von Dihalogenformoximen wird außerdem dadurch erschwert, dass die Bildung des 3-halogensubstituierten 4,5-Dihydroisoxazols bevorzugt in etherischen Lösungsmitteln oder Ethylacetat abläuft, die anschließende Thiocarboxamidinsalz-Bildung andererseits bevorzugt in Alkoholen, Nitrilen oder Ketonen. Das erfordert unter Umständen einen Lösungsmittelwechsel beziehungsweise die Durchführung in getrennten Reaktionsgefäßen.

Aufgabe der vorliegenden Erfindung war demzufolge die Bereitstellung eines kostengünstigen, wirtschaftlichen und sicheren, für die großtechnische Anwendung geeigneten Verfahrens zur Herstellung 5,5-disubstituierter 4,5-Dihydroisoxazol-3-thiocarboxamidinsalze der Formel (I).

Es wurde überraschenderweise gefunden, dass diese Aufgabe durch ein Verfahren gelöst wird, bei man zunächst 3-unsubstituierte, 5,5-disubstituierte 4,5-Dihydroisoxazole der Formel (II) mit halogenierenden Reagenzien in mindestens äquimolarer Menge umsetzt und die dabei erhaltenen 3-halogenierten, 5,5-disubstituierten 4,5-Dihydroisoxazole der Formel (III) mit der mindestens stöchiometrischen Menge Thioharnstoff zu 5,5-disubstituierten 4,5-Dihydroisoxazol-3-thiocarboxamidinsalzen der Formel (I) umsetzt.

Gegenstand der vorliegenden Anmeldung ist daher ein Verfahren zur Herstellung 5,5-disubstituierter 4,5-Dihydroisoxazol-3-thiocarboxamidinsalze der Formel (I), 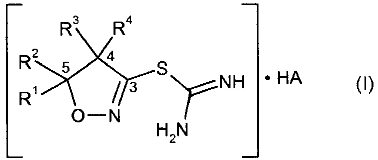 wobei R¹, R², R³, R⁴ und A folgende Bedeutungen haben:
- R¹, R²: unabhängig voneinander jeweils C₁-C₆-Alkyl oder C₁-C₄-Halogenalkyl;
- R³, R⁴: unabhängig voneinander jeweils Wasserstoff, Halogen, C₁-C₆-Alkyl oder C₁-C₄-Halogenalkyl; oder
- R¹ und R², R³ und R⁴ oder R¹ und R³: bilden zusammen eine C₂-C₅-Alkandiyl-Kette, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann; und
- A: Chlor oder Brom;
dadurch gekennzeichnet, dass
i) 3-unsubstituierte 4,5-Dihydroisoxazole der Formel (II) zunächst mit einem Chlorierungs- oder Bromierungsreagenz zu 3-halogenierten 4,5-Dihydroisoxazolen der Formel (III) umgesetzt werden und 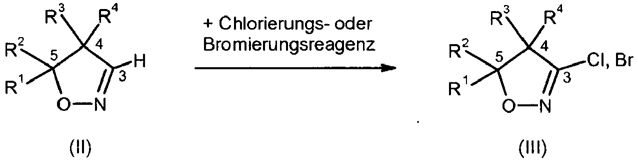
ii) die 3-halogenierten 4,5-Dihydroisoxazole der Formel (III) anschließend mit Thioharnstoff zu den Verbindungen der Formel (I) reagieren, wobei die Schritte i) und ii) im gleichen Lösungsmittel, tert-Butanol oder tert-Amylalkohol, durchgeführt werden.

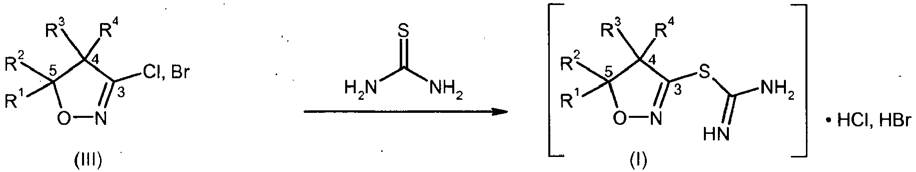

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Die für die Substituenten genannten organischen Molekülteile stellen Sammelbegriffe für individuelle Aufzählungen der einzelnen Gruppenmitglieder dar. Kohlenwasserstoffketten können geradkettig oder verzweigt sein. Sofern nicht anders angegeben tragen halogenierte Substituenten vorzugsweise ein bis fünf gleiche oder verschiedene Halogenatome.

Die Bedeutung Halogen steht jeweils für Fluor, Chlor, Brom oder lod, vorzugsweise für Fluor oder Chlor.

Ferner bedeuten beispielsweise:
Alkyl beziehungsweise die Alkylteile von Carboxyalkyl, Sulfonylalkyl, Phenylalkyl, Aryl-alkyl, Heterocyclylalkyl, Heteroarylalkyl, Alkoxy, Alkylcarbonyl, Hydroxyiminoalkyl, Alkylamino, Alkylcarbonyloxy, Alkylsilyl und Alkylsilyloxy bedeuten eine gesättigte, geradkettige oder verzweigte Kohlenwasserstoffgruppe mit 1 bis 6 beziehungsweise 1 bis 4 Kohlenstoffatomen, beispielsweise Methyl, Ethyl, Propyl, 1-Methylethyl, Butyl, 1-Methylpropyl, 2-Methylpropyl, 1,1-Dimethyl-ethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethyl-ethyl.

Cycloalkyl bezeichnet monocyclische, gesättigte Kohlenwasserstoffgruppen mit drei oder mehr C-Atomen, beispielsweise 3 bis 6 Kohlenstoffringgliedern, wie Cyclopropyl, Cyclobutyl, Cyclopentyl und Cyclohexyl.

Halogenalkyl beziehungsweise die Halogenalkylteile von Haloalkoxy stehen für teilweise oder vollständig halogeniertes Alkyl, wobei das/die Halogenatom(e) insbesondere Fluor, Chlor und/oder Brom ist/sind, beispielsweise Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2-Chlor-2-fluor-ethyl, 2,2,2-Trifluorethyl, 2-Chlor-1,1,2-trifluorethyl, 2-Chlor-2,2-difluorethyl, 2-Brom-2,2-difluorethyl, 2,2-Dichlor-2-fluorethyl, 2,2,2-Trichlorethyl, 1,1,2,2-Tetrafluorethyl, 1,1,2,2-Tetrachlorethyl, Pentafluorethyl, 2,2,3,3-Tetrafluor-1-propyl, 1,1,2,3,3,3-Hexafluor-1-propyl, 1,1,1,3,3,3-Hexafluor-2-propyl, Heptafluor-1-propyl, Heptafluor-2-propyl, 2,2,3,3,4,4,4-Heptafluor-1-butyl oder Nonafluor-1-butyl.

Alkenyl beziehungsweise die Alkenylteile von Phenylalkenyl und Alkenyloxy bedeuten eine einfach ungesättigte, geradkettige oder verzweigte Kohlenwasserstoffgruppe mit zwei bis sechs beziehungsweise zwei bis vier Kohlenstoffatomen und einer Doppelbindung in einer beliebigen Position, beispielsweise Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

Alkinyl beziehungsweise die Alkinylteile von Alkinyloxy bezeichnen geradkettige oder verzweigte Kohlenwasserstoffgruppen mit zwei oder mehr C-Atomen, beispielsweise 2 bis 4, 2 bis 6, oder 3 bis 6 Kohlenstoffatomen und einer Dreifachbindung wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl.

Aryl bezeichnet einen ein- bis dreikernigen aromatischen Carbocyclus mit 6 bis 14 Ringgliedern, beispielsweise Phenyl, Naphthyl und Anthracenyl.

Heteroaryl bezeichnet ein 5- oder 6-gliedriges aromatisches Ringsystem mit ein bis vier Stickstoffatomen oder mit ein bis drei Stickstoffatomen und einem Sauerstoff- oder Schwefelatom, oder mit einem Sauerstoff- oder Schwefelatom.

Heterocyclyl bezeichnet einen gesättigten, partiell ungesättigten oder aromatischen heterocyclischer Ring mit drei oder mehr C-Atomen, z.B. 3-, 4-, 5- oder 6-gliedriger heterocyclischer Ring, der ein bis vier gleiche oder verschiedene Heteroatome, ausgewählt aus der Gruppe Sauerstoff, Schwefel und Stickstoff enthält, und über C oder N gebunden sein kann; wobei ein Schwefel im Heterocyclyl zu S=O oder S(=O)₂ oxidiert sein kann, und wobei mit einem ankondensierten Phenylring oder mit einem C₃-C₆-Carbocyclus oder mit einem weiteren 5- bis 6-gliedrigen Heterocyclus ein bicyclisches Ringsystem ausgebildet werden kann.

Im erfindungsgemäßen Verfahren werden bevorzugt Verbindungen der Formel (II) eingesetzt, deren Variablen folgende Bedeutungen haben und zwar jeweils für sich allein oder in Kombination:
- R¹: C₁-C₆-Alkyl oder C₁-C₄-Haloalkyl ;
- R²: C₁-C₆-Alkyl oder C₁-C₄-Haloalkyl;
- R³: Wasserstoff, Halogen, C₁-C₆-Alkyl, C₁-C₄-Haloalkyl und
- R⁴: Wasserstoff, Halogen, C₁-C₆-Alkyl, C₁-C₄-Haloalkyl, oder R¹ und R², R³ und R⁴
oder R¹ und R³ bilden eine C₂-C₅-Alkandiyl-Kette.

Besonders bevorzugt werden Verbindungen der Formel (II) eingesetzt, deren Variablen folgende Bedeutungen haben und zwar jeweils für sich allein oder in Kombination:
- R¹: C₁-C₄-Alkyl, insbesondere Methyl oder Ethyl, besonders bevorzugt Methyl;
- R²: C₁-C₄-Alkyl, insbesondere Methyl oder Ethyl, besonders bevorzugt Methyl;
- R³: Wasserstoff, Fluor, Chlor, Brom, Methyl, Ethyl, besonders bevorzugt Wasserstoff; und
- R⁴: Wasserstoff, Fluor, Chlor, Brom, Methyl, Ethyl, besonders bevorzugt Wasserstoff.

Außerordentlich bevorzugt wird die Verbindung der Formel (IIa) eingesetzt, deren Variablen folgende Bedeutung haben:
- R¹: Methyl;
- R²: Methyl;
- R³: Wasserstoff;
- R⁴: Wasserstoff.

Im erfindungsgemäßen Verfahren wird die Verbindung der Formel (IIa) zur Verbindung der Formel (Ia) umgesetzt:

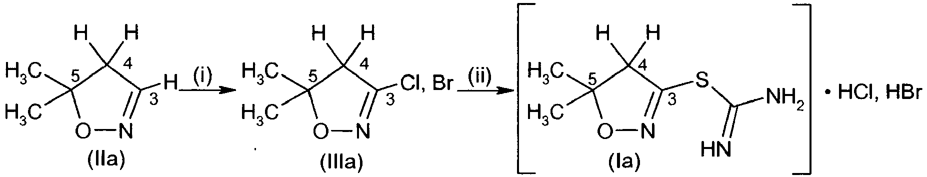

Bestimmte 5,5-disubstituierte 4,5-Dihydroisoxazol-3-thiocarboxamidinsalze der Formel (I) mit R³, R⁴ jeweils unabhängig voneinander in der Bedeutung Wasserstoff, Fluor oder Chlor sind beispielsweise Zwischenprodukte in einem Verfahren zur Herstellung von Oxazol-Herbiziden der Formel (IV), 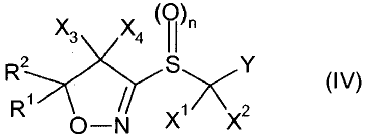 wobei die Variablen folgende Bedeutungen haben:
- n: 0, 1 oder 2;
- X¹, X², X³, X⁴: jeweils unabhängig voneinander Wasserstoff, Fluor oder Chlor; und
- Y: Phenyl, 6-gliedriges Heteroaryl mit ein bis drei Stickstoffatomen oder 5-gliedriges Heteroaryl mit ein bis drei Heteroatomen ausgewählt aus der Gruppe Sauerstoff, Stickstoff und Schwefel, wobei Phenyl und Heteroaryl jeweils durch ein bis fünf Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, Sulfonyl-C₁-C₄-Alkyl, C₂-C₆-Alkenyl, C₂-C₆-Alkinyl, C₁-C₄-Alkoxy, C₁-C₄-Haloalkoxy, C₂-C₆-Alkenyloxy, C₂-C₆-Alkinyloxy und C₁-C₄-Alkylcarbonyloxy, substituiert sein können; und
- R¹, R²: unabhängig voneinander jeweils C₁-C₆-Alkyl oder C₁-C₄-Halogenalkyl; oder R¹ und R² bilden zusammen eine C₂-C₅-Alkandiyl-Kette, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann;

Oxazol-Herbizide der Formel (IV) sind aus WO 02/062770 und WO 01/012613 bekannt.

Das 5,5-disubstituierte 4,5-Dihydroisoxazol-3-thiocarboxamidinsalz der Formel (la) mit R¹ und R² mit der Bedeutung Methyl und R³ und R⁴ mit der Bedeutung Wasserstoff wird bevorzugt als Zwischenprodukt in Verfahren zur Herstellung von Oxazol-Herbiziden der Formel (IVB) verwendet, wobei 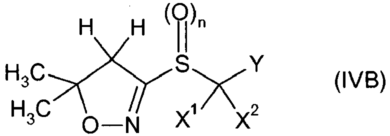
- n: 1 oder 2;
- X¹, X²: jeweils unabhängig voneinander Wasserstoff oder Fluor; und
- Y: Phenyl bedeuten, wobei Phenyl durch ein bis drei Substituenten ausgewählt aus der Gruppe Halogen, C₁-C₄-Alkyl, C₁-C₄-Halogenalkyl und C₁-C₄-Alkoxy substituiert sein kann.

Insbesondere wird das 5,5-disubstituierte 4,5-Dihydroisoxazol-3-thiocarboxamidinsalz der Formel (la) mit R¹ und R² mit der Bedeutung Methyl und R³ und R⁴ mit der Bedeutung Wasserstoff als Zwischenprodukt in Verfahren zur Herstellung auch von Oxazol-Herbiziden der Formel (IVA) verwendet, wobei 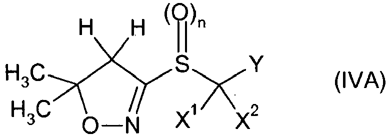
- n: 1 oder 2;
- X¹, X²: jeweils unabhängig voneinander Wasserstoff oder Fluor; und
- Y: Pyrazolyl bedeuten, welches durch ein bis drei Substituenten ausgewählt aus der Gruppe C₁-C₄-Alkyl, C₁-C₄-Halogenalkyl und C₁-C₄-Halogenalkoxy substituiert sein kann.

Außerordentlich bevorzugt wird das 5,5-disubstituierte 4,5-Dihydroisoxazol-3-thiocarboxamidinsalz der Formel (la) mit R¹ und R² mit der Bedeutung Methyl und R³ und R⁴ mit der Bedeutung Wasserstoff als Zwischenprodukt in Verfahren zur Herstellung der Oxazol-Herbizide der Formeln (IVa) und (IVb) verwendet.

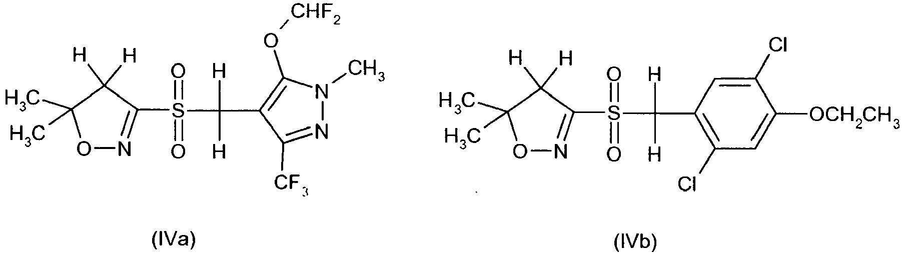

Ausgehend vom Zwischenprodukt der Formel (la) sind die weiteren Schritte im Verfahren beispielsweise zur Herstellung des Oxazol-Herbizids der Formel (IVa) dem Fachmann an sich bereits bekannt oder wurden in der Literatur beschrieben.

Die Darstellung des Pyrazols (c) aus Dicarbonylverbindungen und Hydrazinen wird beispielsweise in JP2007/031342 demonstriert. Die Umsetzung des Hydroxysubstituierten Pyrazols mit Formaldehyd, gefolgt von der Reaktion mit Thiocarboxamidin-Salzen (d) wird in CA 2 560 936 (WO2005/095352) beschrieben. Die Alkylierung der Hydroxygruppe (e) ist dem Fachmann aus JP2007/246396 bekannt. Die abschließende Oxidation des Schwefels zum Sulfon (f) wird beispielsweise in EP 1 405 853 angewandt.

Für die Verbindung der Formel (IVa) lässt sich eine mögliche Synthese wie folgt darstellen:

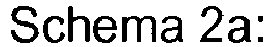

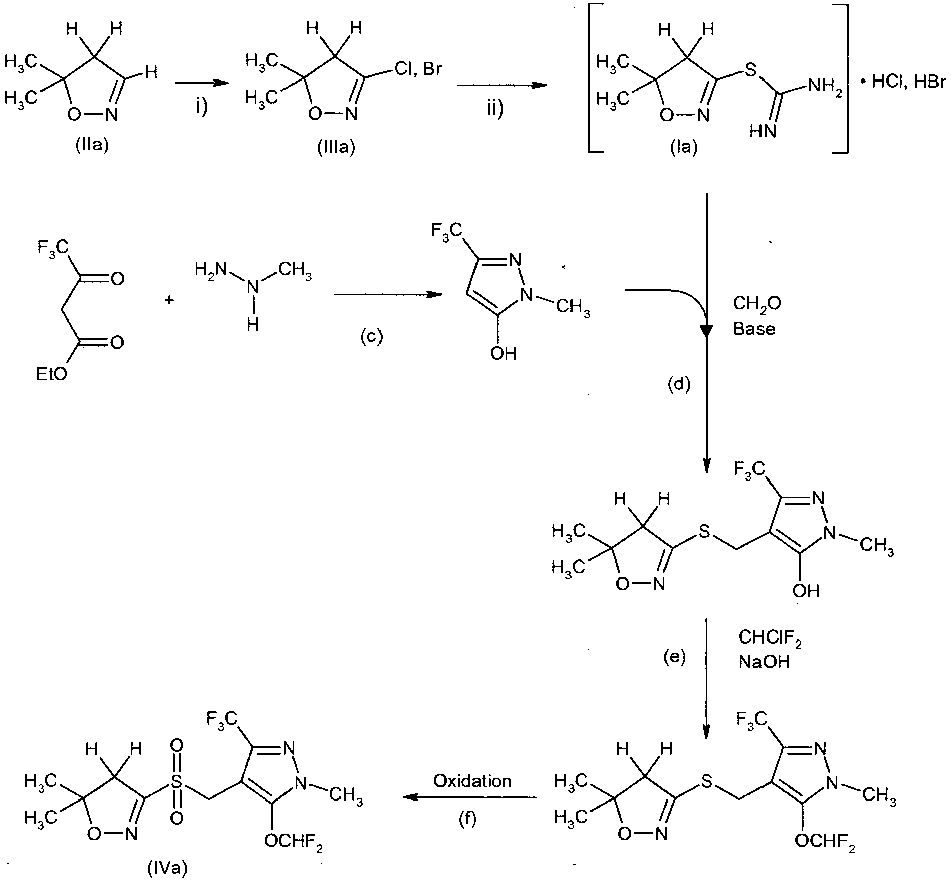

Ausgehend vom Zwischenprodukt der Formel (la) sind die weiteren Schritte im Verfahren beispielsweise zur Herstellung des Oxazol-Herbizids der Formel (IVb) dem Fachmann an sich bereits bekannt oder wurden in der Literatur beschrieben.

Die nucleophile Substitution benzylischer Bromide (g) wurde beispielsweise in WO2007/096576 demonstriert. Die abschließende Oxidation des Schwefels zum Sulfon (h) wird beispielsweise in EP 1 405 853 angewandt.

Für die Verbindung der Formel (IVb) lässt sich eine mögliche Synthese wie folgt darstellen:

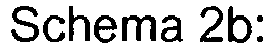

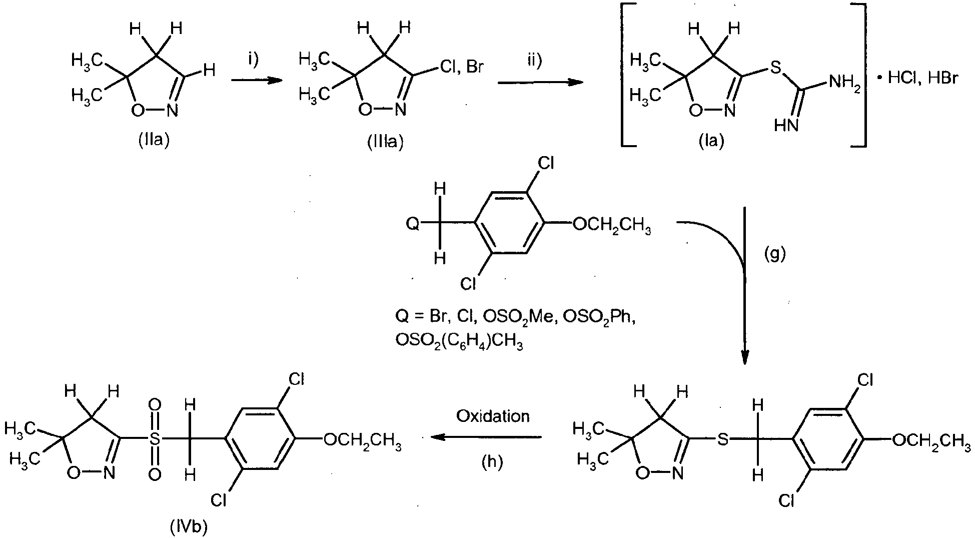

Das erfindungsgemäße Verfahren kann schematisch wie folgt dargestellt werden:

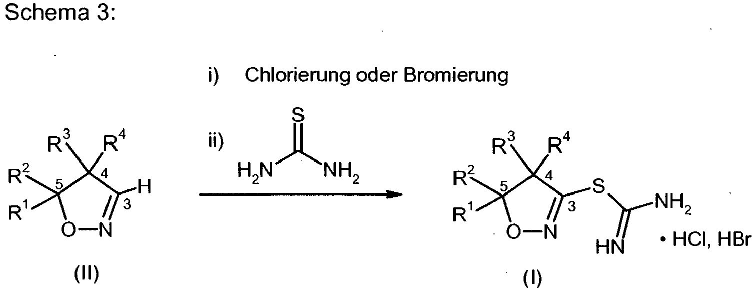

3-unsubstituierte 4,5-Dihydroisoxazole der Formel (II) sind kommerziell erhältlich oder können alternativ gemäß Grünanger, P.; Vita-Finzi, P.: Isoxazoles in Taylor, E. C.: The Chemistry of Heterocyclic Compounds Vol. 49, Wiley&Sons. Inc., New York, 1991, S.460-540 hergestellt werden.

Das erfindungsgemäße Verfahren wird in der Regel unter folgenden Bedingungen durchgeführt:
Die Verbindung der Formel (II) kann als Reinstoff oder als Rohprodukt aus der Bildungsreaktion eingesetzt werden. Dazu wird das 3-unsubstituierte 4,5-Dihydroisoxazol der Formel (II) entweder in einem geeigneten Lösungsmittel gelöst oder suspendiert, oder es liegt aus der Bildungsreaktion in einem geeigneten Lösungsmittel gelöst oder suspendiert vor.

Geeignete Lösungs- beziehungsweise Verdünnungsmittel für die Schritte i) und ii) sind solche, die unter den Reaktionsbedingungen inert sind, beispielsweise Alkohole, chlorierte aromatische oder chlorierte aliphatische Kohlenwasserstoffe, Ester, Ether, Amide, Nitrile oder Gemische dieser Lösungsmittel.
Bevorzugt werden Alkohole, insbesondere tertiäre Alkohohle wie tert-Butanol oder tert-Amylalkohol, chlorierte aromatische oder chlorierte aliphatische Kohlenwasserstoffe wie Chlorbenzol und Chloroform als Lösungsmittel eingesetzt. Außerordentlich bevorzugt wird tert-Butanol verwendet.

Überaschenderweise erweist sich tert-Butanol gegenüber primären aliphatischen Alkoholen als vorteilhaft, da es nur eine vernachlässigbare Tendenz zeigt, 3-Alkoxy-4,5-Dihydroisoxazole zu bilden, die sich in der nachfolgenden Umsetzung mit Thioharnstoff als reaktionsträge herausgestellt haben (Vergleichsbeispiel 1).

Die Reaktionsschritte i) und ii) des erfindungsgemäßen Verfahrens werden im gleichen Lösungs- oder Verdünnungsmittel durchgeführt, in einer besonders bevorzugten Ausführungsform werden beide Schritte, i) und ii), in tert-Butanol durchgeführt.

Die Menge an Lösungs- beziehungsweise Verdünnungsmittel wird in der Regel so gewählt, dass die Reaktionsmischungen während der Umsetzung fließfähig bleiben. In einer bevorzugten Ausführungsform werden weniger als 1 I, besonders bevorzugt weniger als 500 ml Lösungsmittel je Mol des 3-unsubstituierten 4,5-Dihydroisoxazols der Formel (II) eingesetzt. Diese Angaben beziehen sich auf die Gesamtmenge an Lösungsmittel in den Reaktionsstufen i) und ii). Selbstverständlich wird man aus Kostengründen möglichst geringe Mengen an Lösungsmitteln einsetzen. In der Regel wird daher die Lösungsmittelmenge nicht mehr als 5 I je Mol des 3-unsubstituierten 4,5-Dihydroisoxazols der Formel (II) betragen.

Die Umsetzung der Verbindung der Formel (II) zu 3-halogenierten 4,5-Dihydroisoxazolen erfolgt durch Reaktion mit Halogenierungsreagenzien, wobei das Reagenz äquimolar oder im bis zu 10fachen Überschuss eingesetzt wird, bevorzugt in äquimolarer Menge oder im bis zu 1,5fachen Überschuss.

Geeignete Reagenzien sind beispielsweise elementares Chlor oder Brom, wässrige Lösungen von Hypochlorit, Hypobromit, Lösungen anorganischer Bromide und Chloride wie HCl, HBr, NaCl, KCI, NaBr oder KBr in Gegenwart eines Oxidationsmittels wie Wasserstoffperoxid, Kaliumperoxomonosulfat oder organischen Peroxiden wie tert-Butylhydroperoxid, die Kombination aus Brom und Wasserstoffperoxid, organische Hypochlorite wie tert-Butylhypochlorit, Trichlorisocyanursäure, Thionylchlorid oder Sulfurylchlorid, N-Bromsuccinimid, N-Chlorsuccinimid oder 1,3-Dibrom-5,5-dimethylhydantoin.
Die halogenierenden Reagenzien können als Reinstoff oder als Lösung in einem der oben angegebenen Lösungsmittel eingesetzt werden.
Besonders bevorzugt sind die Halogene Brom und Chlor.

In einer außerordentlich bevorzugten Ausführungsform wird Chlorgas in die Reaktionslösung eingeleitet bis das Startmaterial vollständig umgesetzt ist. Der Reaktionsverlauf kann mittels dem Fachmann bekannter Verfahren, beispielsweise durch HPLC-Analyse des entstandenen Reaktionsproduktes verfolgt werden.

Die Umsetzung erfolgt bei Temperaturen zwischen -30°C und 150°C, bevorzugt bei Temperaturen zwischen -10°C und 30°C.

Das Reaktionsprodukt aus Schritt i), 3-halogeniertes 4,5-Dihydroisoxazol der Formel (III), kann im erfindungsgemäßen Verfahren direkt ohne Aufreinigung oder nach einer Aufreinigung in Schritt ii) mit Thioharnstoff zu 4,5-Dihydroisoxazol-3-thiocarboxamidinsalzen der Formel (I) umgesetzt werden.

Eine Aufreinigung des Reaktionsproduktes aus Schritt i), 3-halogeniertes 4,5-Dihydroisoxazol der Formel (III), kann in an sich üblicher Weise erfolgen, beispielsweise durch Destillation, Kristallisation, Ausfällen, Extraktion oder Chromatographie, bevorzugt durch Destillation.

In einer bevorzugten Ausführungsform des erfindungsgemäßen Verfahrens wird das Reaktionsprodukt aus Schritt i), 3-halogeniertes 4,5-Dihydroisoxazol der Formel (III), direkt ohne Aufreinigung in Schritt ii) mit Thioharnstoff zu 4,5-Dihydroisoxazol-3-thiocarboxamidinsalzen der Formel (I) umgesetzt.

In einer besonders bevorzugten Ausführungsform des erfindungsgemäßen Verfahrens wird das Reaktionsprodukt aus Schritt i), 3-halogeniertes 4,5-Dihydroisoxazol der Formel (III), direkt ohne Aufreinigung in Schritt ii) mit Thioharnstoff zu 4,5-Dihydroisoxazol-3-thiocarboxamidinsalzen der Formel (I) im selben Reaktionsgefäß, also in einem Ein-Topf-Verfahren umgesetzt.

Nach der Halogenierung in Schritt i), gegebenenfalls einer Aufreinigung des Reaktionsproduktes, Spülen des Reaktionsgefäßes mit Stickstoff oder Anlegen eines Vakuums, wird die Reaktion zu den 5,5-disubstituierten 4,5-Dihydroisoxazol-3-thiocarboxamidinsalzen der Formel (I) durch Zugabe von Thioharnstoff durchgeführt.

In der Regel wird Thioharnstoff in Schritt ii) als Feststoff portionsweise zugegeben, bis die Reaktion abgeschlossen ist. Bevorzugt wird Thioharnstoff in äquimolarer Menge oder im bis zu 1,2fachen Überschuss eingesetzt, bezogen auf das in der Reaktionsmischung vorliegende 3-halogenierte 4,5-Dihydroisoxazol.

Die Reaktion wird bei Temperaturen zwischen 0°C und 100°C, bevorzugt zwischen Raumtemperatur und 50°C durchgeführt.

Überraschenderweise erfolgt die Umsetzung mit Thioharnstoff in Schritt ii) des erfindungsgemäßen Verfahrens in tert-Butanol rasch in glatter Reaktion auch ohne zusätzlich zugegebene Säure. In EP 1 829 868 wird in einem Vergleichsbeispiel bei der Umsetzung von isoliertem 3-Chlor-5,5-dimethyl-4,5-Dihydroisoxazol mit Thioharnstoff in Ethanol ohne Säurezugabe das gewünschte Thiocarboxamidinsalz nach 10 h bei 30 °C mit nur 10% Ausbeute erhalten.
Das erfindungsgemäße Verfahren zur Herstellung 5,5-disubstituierter 4,5-Dihydroisoxazol-3-thiocarboxamidinsalze liefert die entsprechenden 5,5-disubstituierten 4,5-Dihydroisoxazol-3-thiocarboxamidinsalze in sehr hohen Ausbeuten. Eine zusätzliche Säurezugabe ist im erfindungsgemäßen Verfahren nicht erforderlich.

Die Aufarbeitung der in Schritt ii) erhaltenen Reaktionsmischung erfolgt in an sich üblicher Weise. Beispielsweise kann das Produkt des erfindungsgemäßen Verfahrens, 5,5-disubstituiertes 4,5-Dihydroisoxazol-3-thiocarboxamidinsalz der Formel (I), nach Entfernen des Lösungsmittels durch Umkristallisation gereinigt werden. Gemäß einer bevorzugten Ausführungsform fällt das Produkt in kristalliner Form, gegebenenfalls nach Konzentration der Reaktionslösung oder Zugabe eines Antisolvens, beispielsweise einem unpolaren Lösungsmittel wie Aceton oder Toluol, aus dem Reaktionsmedium aus.

In einer Ausführungsform des erfindungsgemäßen Verfahrens wird das 3-unsubstituierte 5,5-disubstituierte 4,5-Dihydroisoxazol der Formel (IIb) wobei R¹ und R² C₁-C₆-Alkyl oder C₁-C₄-Halogenalkyl bedeuten; oder R¹ und R² zusammen eine C₂-C₅-Alkandiyl-Kette bilden, die ein- bis vierfach durch C₁-C₄-Alkyl substituiert sein kann und/oder durch Sauerstoff oder durch gegebenenfalls C₁-C₄-Alkyl-substituierten Stick-stoff unterbrochen sein kann; und R³ und R⁴ Wasserstoff bedeuten;
in einer Vorstufe hergestellt und, gegebenenfalls nach Lösungsmittelwechsel, direkt im erfindungsgemäßen Verfahren weiter eingesetzt.

Die Vorstufe des erfindungsgemäßen Verfahrens ist ein Verfahren zur Herstellung 5,5-disubstituierter 4,5-Dihydroisoxazole der Formel (IIb), 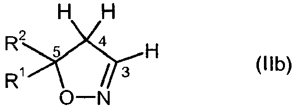 wobei
- R¹, R²: unabhängig voneinander jeweils C₁-C₆-Alkyl oder C₁-C₄-Halogenalkyl bedeuten; oder R¹ und R² zusammen eine C₂-C₅-Alkandiyl-Kette bilden, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann;
dadurch gekennzeichnet, dass ein Oxim der Formel (AII) 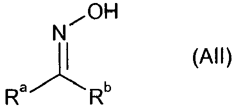 wobei
- R^{a}, R^{b}: unabhängig voneinander jeweils Wasserstoff, C₁-C₆-Alkyl, C₁-C₄-Alkylcarbonyl, Hydroxyimino-C₁-C₄-alkyl, Phenyl, Phenyl-C₁-C₄-Alkyl oder Phenyl-C₂-C₄-Alkenyl bedeuten, wobei die Phenylringe ein- oder mehrfach durch C₁-C₄-Alkyl, C₁-C₄-Alkoxy, Di-C₁-C₄-Alkylamino, Halogen, Hydroxy oder Nitro substituiert sein können; oder R^{a} und R^{b} zusammen eine C₂-C₅-Alkandiyl-Kette bilden;
mit einer Carbonylverbindung der Formel (AIII), 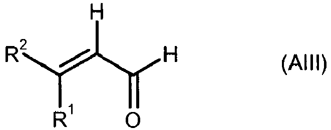 wobei R¹ und R² die oben genannten Bedeutungen haben;
in Anwesenheit eines Katalysators und gegebenenfalls in Anwesenheit eines Lösungsmittels umgesetzt wird.

Für die Vorstufe des erfindungsgemäßen Verfahrens werden bevorzugt Verbindungen der Formeln (AII) und (AIII) eingesetzt, wobei die Variablen jeweils für sich allein oder in Kombination folgende Bedeutungen haben:
- R¹: C₁-C₆-Alkyl oder C₁-C₄-Halogenalkyl;
- R²: C₁-C₆-Alkyl oder C₁-C₄-Halogenalkyl;
- R^{a}: Wasserstoff, C₁-C₆-Alkyl, C₁-C₄-Alkylcarbonyl, Hydroxyimino-C₁-C₄-alkyl; und
- R^{b}: C₁-C₆-Alkyl; oder R^{a} und R^{b} bilden eine C₂-C₅-Alkandiyl-Kette.

Besonders bevorzugt werden Verbindungen der Formeln (All) und (AIII) eingesetzt, wobei die Variablen jeweils für sich allein oder in Kombination folgende Bedeutungen haben:
- R¹: C₁-C₄-Alkyl, insbesondere Methyl oder Ethyl, besonders bevorzugt Methyl;
- R²: C₁-C₄-Alkyl, insbesondere Methyl oder Ethyl, besonders bevorzugt Methyl;
- R^{a}: Wasserstoff, C₁-C₄-Alkyl, insbesondere Methyl, Ethyl, Isopropyl oder Isobutyl, besonders bevorzugt Methyl und Ethyl; und
- R^{b}: C₁-C₄-Alkyl, insbesondere Methyl oder Ethyl; oder R^{a} und R^{b} bilden eine C₂-C₅-Alkandiyl-Kette.

Außerordentlich bevorzugt werden Verbindungen der Formeln (AII) und (AIII) eingesetzt, wobei die Variablen jeweils für sich allein oder in Kombination folgende Bedeutungen haben:
- R¹: Methyl;
- R²: Methyl;
- R^{a}: Methyl oder Ethyl;
- R^{b}: Methyl oder Ethyl.

Für die Vorstufe des erfindungsgemäßen Verfahrens werden das Oxim der Formel (AII) mit der Carbonylverbindung der Formel (AIII) in Anwesenheit eines Säurekatalysators oder eines Säure-Base-Katalysators und gegebenenfalls in Anwesenheit eines organischen Lösungsmittels umgesetzt (Schema 4):

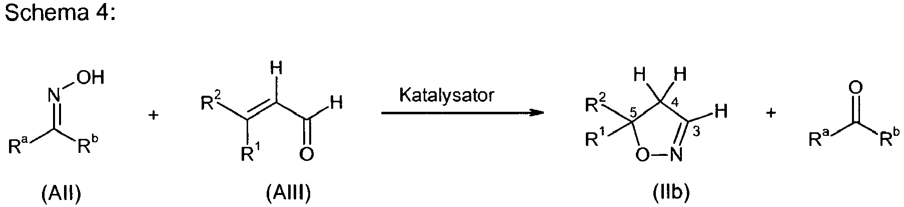

Die Oxime der Formel (AII) sind entweder kommerziell erhältlich oder können beispielsweise gemäß Yamane, M.; Narasaka, K., in Science of Synthesis, 27 (2004), S.605 hergestellt werden.

Die Carbonylverbindungen der Formel (AIII) sind ebenfalls kommerziell erhältlich oder können beispielsweise gemäß Escher, I.; Glorius, F., in Science of Synthesis, 25 (2006), S.733 hergestellt werden.

In der Regel wird das erfindungsgemäße Verfahren zur Herstellung der Verbindung der Formel (IIb) unter folgenden Bedingungen durchgeführt:
Das Oxim der Formel (AII) und die Carbonylverbindung der Formel (AIII) werden im erfindungsgemäßen Verfahren in einem Molverhältnis von 3:1 bis 1:3 eingesetzt. Bevorzugt beträgt der Überschuss einer der beiden Komponenten bis zu 20 mol%, insbesondere der Carbonylverbindung der Formel (AIII). Das bevorzugte Molverhältnis des Oxims (AII) zur Carbonylverbindung (AIII) liegt entsprechend bei 1.0:0.8 bis 1.0:1.2, besonders bevorzugt bei etwa 1.0:1.0 bis 1.0:1.1.

Die Umsetzung des Oxims der Formel (AII) und der Carbonylverbindung der Formel (AII) findet in Gegenwart eines Katalysators statt. Geeignete Katalysatoren sind bestimmte Säuren (Säurekatalysator) oder Mischungen bestimmter Säuren und bestimmter Basen (Säure-Base-Katalysator).

Das erfindungsgemäße säurekatalysierte Verfahren kann schematisch wie folgt dargestellt werden:

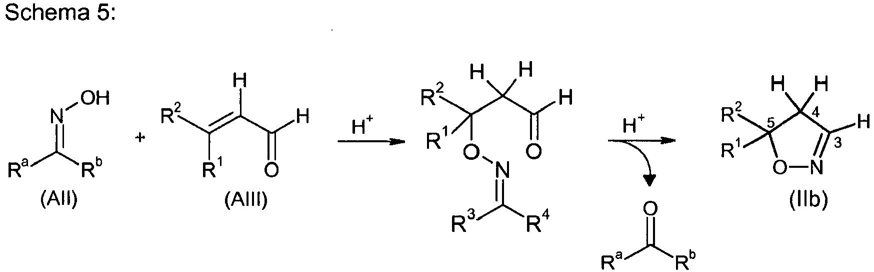

Geeignete Säurekatalysatoren sind Protonendonatoren (Brönstedt-Säuren), beispielsweise anorganische und organische Säuren. Beispiele für anorganische Säuren sind Halogenwasserstoffsäuren und Sauerstoffsäuren, insbesondere Salzsäure, Schwefelsäure, Salpetersäure, Phosphorsäure, Phosphonsäure und Phosphinsäure.

Beispiele für organische Säuren sind aliphatische und aromatische Säuren wie Alkylsulfonsäuren, Arylsulfonsäuren, Mono-C₁-C₆-alkylphosphate, Di-C₁-C₆-alkylphosphate, Monoarylphosphate, Diarylphosphate, Alkylcarbonsäuren, Halogenalkylcarbonsäuren und Heterocyclylcarbonsäuren, insbesondere Methansulfonsäure, p-Toluolsulfonsäure, Zitronensäure, Trifluoressigsäure und Prolin.

Im allgemeinen läuft die Reaktion säurekatalysiert mit solchen Säuren in guter Ausbeute ab, deren pKs-Wert kleiner als 3.5 ist.

Wird das erfindungsgemäße Verfahren nur unter Säurekatalyse durchgeführt, so werden bevorzugt starke Säuren wie Phosphorsäure, Mono-C₁-C₆-alkylphosphate, Di-C₁-C₆-alkylphosphate, Monoarylphosphate, Diarylphosphate, Schwefelsäure, Sulfonsäuren oder Trifluoressigsäure verwendet.

Das erfindungsgemäße säure-base-katalysierte Verfahren könnte nach folgendem Schema ablaufen:

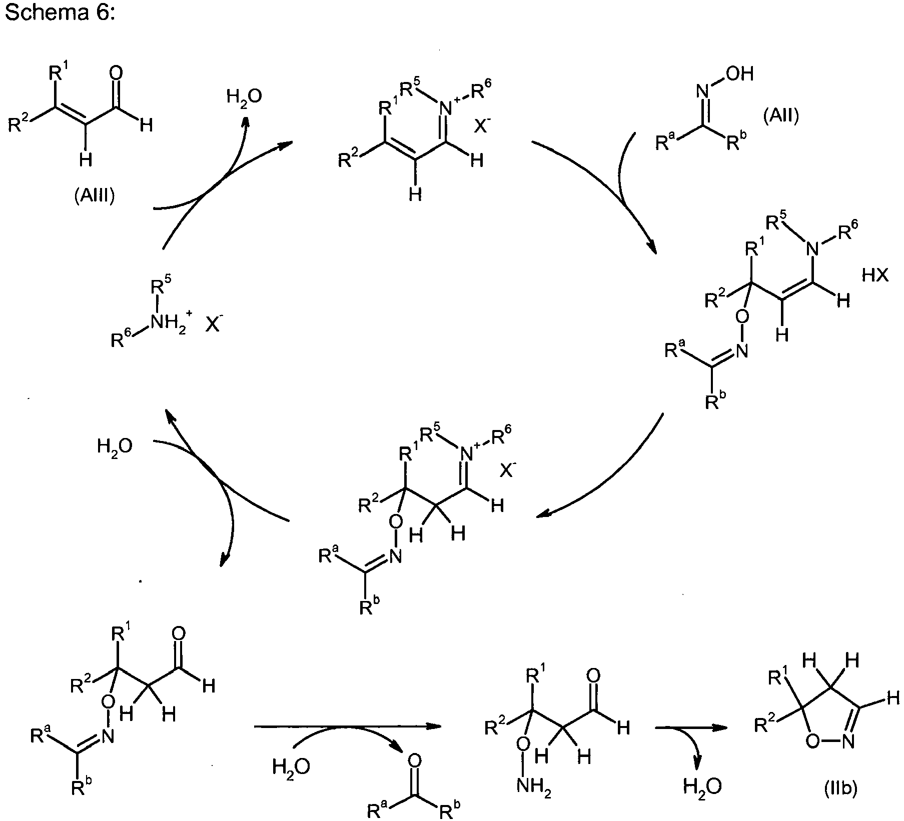

Geeignete Säure-Base-Katalysatoren sind Mischungen aus den oben beschriebenen Säuren und bestimmten Basen, wobei Säure und Base getrennt voneinander oder als Säureadditionssalz eingesetzt werden können.

Als geeignete Basen haben sich Verbindungen erwiesen, die ein oder mehrere Heteroatome enthalten, beispielsweise Stickstoff, Sauerstoff, Schwefel oder Phosphor, wobei Stickstoff ein bevorzugtes Heteroatom ist.

Beispiele für solche Basen sind primäre oder sekundäre Amine der Formel (V) 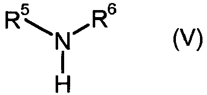 wobei R⁵ und R⁶ unabhängig voneinander jeweils C₁-C₆-Alkyl, C₃-C₆-Cycloalkyl, C₂-C₆-Alkenyl, C₂-C₆-Alkinyl, Tri-C₁-C₆-Alkylsilyl, Aryl, Aryl-C₁-C₆-Alkyl, Heteroaryl, Heteroaryl-C₁-C₆-Alkyl bedeuten, und wobei die Aryl und Heteroarylteile der Substituenten selbst durch ein bis drei Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, C₂-C₆-Alkenyl, C₂-C₆-Alkinyl, C₁-C₄-Alkoxy, C₁-C₄-Haloalkoxy, C₂-C₆-Alkenyloxy, C₂-C₆-Alkinyloxy, C₁-C₄-Alkylcarbonyloxy substituiert sein können;
und wobei R⁵ zusätzlich Wasserstoff bedeuten kann.

Bevorzugt sind Verbindungen der Formel (V), in denen R⁵ und R⁶ unabhängig voneinander jeweils C₁-C₆-Alkyl, C₃-C₆-Cycloalkyl, Tri-C₁-C₆-Alkylsilyl, Aryl oder Aryl-C₁-C₆-Alkyl bedeuten; und wobei die Arylteile der Substituenten selbst durch ein bis drei Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, C₂-C₆-Alkenyl, C₂-C₆-Alkinyl, C₁-C₄-Alkoxy, C₁-C₄-Haloalkoxy, C₂-C₆-Alkenyloxy, C₂-C₆-Alkinyloxy, C₁-C₄-Alkylcarbonyloxy substituiert sein können;
und wobei R⁵ zusätzlich Wasserstoff bedeuten kann.

Besonders bevorzugt sind Verbindungen der Formel (V), in denen R⁵ und R⁶ unabhängig voneinander jeweils Methyl, Ethyl, Propyl, 1-Methylethyl, Butyl, Cyclopentyl, Cyclohexyl, Phenyl, Naphthyl, Benzyl oder Trimethylsilyl bedeuten; und wobei R⁵ zusätzlich Wasserstoff bedeuten kann; beispielsweise N-Methylanilin.

Alternativ können R⁵ und R⁶ gemeinsam eine Ringstruktur der Formel (VI) bilden, 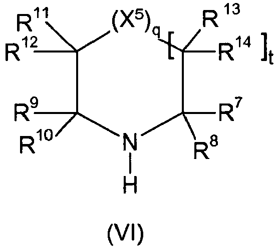 wobei
X⁵ für O, S, NR¹⁵ oder CR¹⁶R¹⁷ steht;
q 0 oder 1 ist;
t 0 oder 1 ist; und
R⁷, R⁸, R⁹, R¹⁰, R¹¹, R¹², R¹³, R¹⁴, R¹⁶, R¹⁷ unabhängig voneinander ausgewählt sind aus Wasserstoff, Hydroxy, Carboxy, Amino, Nitro, Aminocarbonyl, C₁-C₆-Alkyl, C₁-C₆-Alkoxy, C₁-C₆-Alkylcarbonyl, Mono-C₁-C₆-alkylamino, Di-C₁-C₆-alkylamino, Aryl, Heteroaryl, Aryl-C₁-C₆-alkoxy, und wobei C₁-C₆-Alkyl selbst durch Aryl, Heteroaryl, Heterocyclyl oder Trimethylsilyloxy substituiert sein kann und wobei die Aryl, Heterocyclyl und Heteroarylteile der Substituenten selbst durch ein bis drei Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, C₂-C₆-Alkenyl, C₂-C₆-Alkinyl, C₁-C₄-Alkoxy, C₁-C₄-Haloalkoxy, C₂-C₆-Alkenyloxy, C₂-C₆-Alkinyloxy und C₁-C₄-Alkylcarbonyloxy substituiert sein können;
oder R¹¹ und R¹² und/oder R¹³ und R¹⁴ bilden gemeinsam mit dem Kohlenstoffatom, an das sie gebunden sind, eine Ketogruppe; und R¹⁵ ist ausgewählt aus Wasserstoff, C₁-C₆-Alkyl, Aryl-C₁-C₆-alkyl.

Bevorzugt sind Amine der Formel (V) beziehungsweise (VI), wobei R⁵ und R⁶ gemeinsam mit der NH-Gruppe einen Imidazolin-5-on Ring der Formel (VII) bilden, 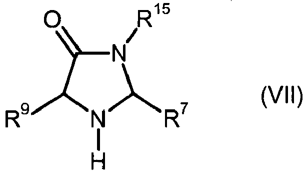 wobei die Substituenten die oben genannte Definition haben.

Besonders bevorzugt sind hierbei Verbindungen, bei denen R⁷ und R⁹ unabhängig voneinander ausgewählt sind aus C₁-C₆-Alkyl und Aryl-C₁-C₆-alkyl, vorzugsweise aus Methyl, Ethyl, 1-Methylethyl, 1,1-Dimethylethyl und Phenylmethyl.

In der Regel werden die Katalysatoren der Reaktionsmischung in katalytischer Menge zugesetzt. In einer erfindungsgemäßen Ausführungsform liegt das Molverhältnis der Verbindung der Formel (All) zu den Säurekatalysatoren beziehungsweise zu den Säure-Base-Katalysatoren unter 1:0.1. In einer bevorzugten Ausführungsform liegt das Molverhältnis unter 1:0.05, in einer besonders bevorzugten Ausführungsform unter 1:0.02.

Das Oxim der Formel (AII) und die Carbonylverbindung der Formel (AIII) können erfindungsgemäß sowohl ohne Zugabe eines Lösungsmittels als auch unter Zugabe eines geeigneten Lösungsmittels umgesetzt werden.

Gemäß einer Ausführungsform des erfindungsgemäßen Verfahrens werden das Oxim der Formel (All) und die Carbonylverbindung der Formel (AIII) unter Zugabe eines Lösungsmittels miteinander umgesetzt

Die Menge an Lösungs- beziehungsweise Verdünnungsmittel wird in der Regel so gewählt, dass die Reaktionsmischungen während der Umsetzung fließfähig bleiben.

In einer bevorzugten Ausführungsform des erfindungsgemäßen Verfahrens beträgt der Anteil des Lösungsmittels im Reaktionsansatz, also vor Reaktionsbeginn, weniger als 80 Gew.%.

Geeignete Lösungsmittel sind organische Lösungsmittel, beispielsweise aromatische Kohlenwasserstoffe wie Toluol, o-, m-, p-Dimethylbenzol, 1,3,5-Trimethylbenzol, Ethylbenzol, Chlorbenzol, o-,m-,p-Dichlorbenzol, halogenierte aliphatische Kohlenwasserstoffe wie Tetrachlorethan, Trichlormethan, Dichlormethan und Dichlorethen, aliphatische Kohlenwasserstoffe wie Pentan, Hexan, Heptan, Octan, Cyclopentan, Methylcyclopentan und Cyclohexan, Ether wie Diethylether, Methyl-tert.-butylether, Tetrahydrofuran und Dioxan, Alkohole wie Methanol, Ethanol, Propanol, tertiäre Alkohole wie tert-Butanol und tert.-Amylalkohol, Ester wie Essigsäureethylester, Nitrile wie Acetonitril oder Mischungen der genannten Lösungsmittel.

Bevorzugte Lösungsmittel sind aromatische Kohlenwasserstoffe, halogenierte aliphatische Kohlenwasserstoffe, aliphatische Kohlenwasserstoffe, Ether und Alkohole.

Besonders bevorzugte Lösungsmittel sind aromatische Kohlenwasserstoffe, insbesondere Toluol, Chlorbenzol, o-,m-Dichlorbenzol, tertiäre Alkohole wie tert-Butanol und tert-Amylalkohol und Mischungen dieser Lösungsmittel.

Entsprechend der Erfindung wird das Verfahren zur Herstellung des 3-unsubstituierten 5,5-disubstituierten 4,5-Dihydroisoxazols der Formel (II) im gleichen Lösungsmittel durchgeführt wie die anschließende Halogenierung und Thiocarboxamidinsalz-Bildung zur Herstellung des 5,5-disubstituierten 4,5-Dihydroisoxazol-3-thiocarbamidinsalzes der Formel (I) insbesondere in tert-Butanol.

In der Regel kommt es nicht darauf an, in welcher Reihenfolge das Oxim der Formel (AII), die Carbonylverbindung der Formel (AIII), der Katalysator und gegebenenfalls das Lösungsmittel in das Reaktionsgefäß vorgelegt beziehungsweise zugegeben werden.
Gemäß einer Ausführungsform der Erfindung werden das Oxim der Formel (All) und die Carbonylverbindung der Formel (AIII) und gegebenenfalls das Lösungsmittel vorgelegt und die gewünschte Temperatur eingestellt. Anschließend wird der Katalysator zugegeben.
Gemäß einer anderen Ausführungsform der Erfindung werden das Oxim der Formel (AII), der Katalysator und gegebenenfalls das Lösungsmittel vorgelegt und die gewünschte Temperatur eingestellt. Anschließend wird die Carbonylverbindung der Formel (AIII) zugegeben.

Unter Zugabe versteht man dabei sowohl die portionsweise als auch kontinuierliche Zugabe eines Stoffes. Die Zugabe des Katalysators beziehungsweise der Carbonylverbindung der Formel (AIII) erfolgt vorzugsweise ohne Lösungsmittel oder gelöst in einem wie oben definierten organischen Lösungsmittel im Verlauf der Reaktion.

Normalerweise arbeitet man bei einer Reaktionstemperatur von -40 bis 100°C, vorzugsweise von -20 bis 60°C, insbesondere von 0 bis 30°C.

Das Reaktionsgemisch kann ohne weitere Aufarbeitung oder nach Entfernung der entstandenen Carbonylverbindung direkt anderen Verfahren zugeführt werden. Die Entfernung der Carbonylverbindung erfolgt mit dem Fachmann bekannten Mitteln, beispielsweise durch Destillation oder Filtration. Das Reaktionsprodukt, das 5,5-disubstituierte 4,5-Dihydroisoxazol der Formel (II), kann auch aus dem Reaktionsgemisch abgetrennt werden, beispielsweise durch direkte Destillation, Extraktion oder Chromatographie, bevorzugt durch Destillation.

In einer bevorzugten Ausführungsform des erfindungsgemäßen Verfahrens wird das Reaktionsprodukt der Vorstufe, das 3-unsubstituierte 5,5-disubstituierte 4,5-Dihydroisoxazol der Formel (IIb) nach destillativer Entfernung der entstandenen Carbonylverbindung direkt weiter in Schritt i) des erfindungsgemäßen Verfahrens zur Herstellung der Verbindung der Formel (I) eingesetzt.

### Ausführungsbeispiele:

### Beispiel 1: (Säure-Base Katalyse)

### Synthese von 5,5-Dimethyl-4,5-Dihydroisoxazol

10,0 g (0,137 mol; 100 mol%) Acetonoxim wurden mit 12,7 g (0,151 mol, 110 mol%) 3-Methyl-2-butenal versetzt und auf 10 °C gekühlt. Zu dieser Mischung wurde im Verlauf von 3 h portionsweise eine Mischung von 0,13 g (1,2 mmol, 0,9 mol%) N-Methylanilin und 0,14 g (1,2 mmol, 0,9 mol%) Trifluoressigsäure gegeben, und die Temperatur nach 20% der Zugabe auf 22 °C erhöht. Nach 3 h wurde das Wertprodukt aus der Reaktionsmischung durch fraktionierte Destillation im Vakuum isoliert. Siedepunkt 44-48 °C bei 17-18 mbar. Es wurden 10,0 g 5,5-Dimethyl-4,5-Dihydroisoxazol erhalten, gemäß ¹H-NMR 89%ig (66%).
¹H-NMR (CDCl₃): 1,40 (s, 6H), 2,75 (d, 2H), 7,06 (br s, 1 H).

### Beispiel 2: (Säure-Base Katalyse)

### Synthese von 5,5-Dimethyl-4,5-Dihydroisoxazol

50,0 g (0,684 mol, 100 mol%) Acetonoxim wurden mit 62,6 g (0,744 mol, 109 mol%) 3-Methyl-2-butenal versetzt und auf 10-15 °C gekühlt. Zu dieser Mischung wurden im Verlauf von 48 h 1,5 g (6,8 mmol, 1 mol%) N-Methylanilinium-Trifluoracetat in 0,1 g Portionen gegeben. Das Wertprodukt wurde anschließend aus der Reaktionsmischung durch fraktionierte Destillation im Vakuum isoliert. Siedepunkt 44-48 °C bei 17-18 mbar. Es wurden 56,9 g 5,5-Dimethyl-4,5-Dihydroisoxazol erhalten, gemäß ¹H-NMR >91 %ig (76%).
¹H-NMR (CDCl₃): 1,40 (s, 6H), 2,75 (d, 2H), 7,06 (br s, 1 H).

### Beispiel 3: (Säure-Katalyse)

### Synthese von 5,5-Dimethyl-4,5-Dihydroisoxazol

4,35 g Acetonoxim (59,5 mmol, 100 mol%) und 5,27 g 3-Methyl-2-butenal (62,6 mmol, 105 mol%) wurden gemischt und mit 0,13 g Trifluoressigsäure (1,1 mmol, 1,9 mol%) versetzt. Es wurde 60 h bei Raumtemperatur gerührt und das Produkt im Vakuum destilliert (46°C, 18 mbar). Es wurden 4,7 g farbloses Öl erhalten, laut NMR mit einer Reinheit >95% (45,0 mmol, 76%).

### Beispiel 4: (Säure-Base Katalyse)

### Synthese von 5,5-Dimethyl-4,5-Dihydroisoxazol

0,4 g (2S,5S)-2-tert-Butyl-3-methyl-5-benzyl-4-imidazolinon (1,6 mmol, 1 mol%) wurde in 50 ml n-Pentan vorgelegt und bei 0 °C mit 0,19 g Trifluoressigsäure (1,6 mmol, 1 mol%) versetzt. Es wurde 30 min bei -3 - 0°C gerührt. Zu der entstandenen Suspension wurden bei 0 °C 11,9 g Acetonoxim (0,163 mol, 100 mol%) gegeben, die Mischung wurde auf 20 °C erwärmt, und 16,5 g 3-Methyl-2-butenal (0,196 mol, 120 mol%) innerhalb von 5 min zugetropft. Es wurde 16 h bei dieser Temperatur gerührt, und das Produkt durch Destillation isoliert. Es wurden 15,5 g 5,5-Dimethyl-4,5-Dihydroisoxazol mit einer Reinheit (GC) von 88%, entsprechend einer Ausbeute von 84% erhalten.

### Beispiel 5: Synthese von 3-Chlor-4,5-dihydro-5,5-dimethylisoxazol

0,61 g (6,2 mmol, 100 mol%) 4,5-Dihydro-5,5-dimethylisoxazol wurden in 15 ml Tetrachlorkohlenstoff gelöst. Es wurde bei 24-33 °C Chlorgas eingeleitet bis das Edukt vollständig abreagiert hatte. Das Lösungsmittel wurde bei 25 °C durch einen Stickstoffstrom vertrieben. Man erhielt 0,67 g 3-Chlor-4,5-dihydro-5,5-dimethylisoxazol als farbloses Öl, laut GC und NMR mit einer Reinheit von 90%, Ausbeute 75%.
¹H-NMR (CDCl₃): 1,46 (s, 6H), 2,93 (s, 2H).

### Beispiel 6: Synthese von [5,5-Dimethyl(4,5-dihydroisoxazol-3-yl)]thiocarboxamidin Hydrochlorid mit destilliertem Edukt

14,4 g (90%ig laut HPLC, 130 mmol, 100 mol%) 4,5-Dihydro-5,5-dimethylisoxazol wurden in 20 g tert-Butanol gelöst. Im Verlauf von einer Stunde wurden bei 22-27 °C 12 g (170 mol, 130 mol%) Chlor getaucht eingegast und die Lösung anschließend eine Stunde mit Stickstoff gespült. 7,1 g (93 mmol, 72 mol%) Thioharnstoff wurden als Feststoff zugegeben und die Reaktionsmischung drei Stunden bei 20 °C gerührt. Zur Vervollständigung der Reaktion wurden 1,0 g (13 mmol, 10 mol%) Thioharnstoff zugegeben und die Mischung 60 h Stunden gerührt. Das Lösungsmittel wurde unter vermindertem Druck entfernt und der Rückstand in Petrolether suspendiert. Der Rückstand wurde abgesaugt und getrocknet; Auswage 26 g. Eine 10 g Probe wurde aus Isopropanol umkristallisiert. Man erhielt 6,2 g des Produkts als farblosen Feststoff, Ausbeute 59%. Schmelzpunkt 138-140 °C.

### Beispiel 7: Synthese von [5,5-Dimethyl(4,5-dihydroisoxazol-3-yl)]thiocarboxamidin Hydrochlorid ausgehend von 3-Methyl-2-butenal

41,1 g (0,56 mol, 100 mol%) Acetonoxim wurden in 150 mL Chlorbenzol gelöst und mit 1,0 g N-Methylanilinium-Triflat (4,5 mmol, 0,8 mol%) versetzt. Im Verlauf von drei Stunden wurden 48,7 g (0,58 mol, 103 mol%) 3-Methyl-2-butenal bei einer Temperatur von 20-23 °C zugegeben. Es wurde 16 h bei dieser Temperatur gerührt und das bei der Reaktion entstandene Aceton abdestilliert (50 °C, 120 mbar). 93 g (1,31 mol, 230 mol%) Chlor wurden bei 0-5 °C im Verlauf von 5 h eingegast. Die Lösung wurde mit Stickstoff gespült, 200 mL Ethanol zugegeben und die Temperatur auf 45-50 °C erhöht. 24,4 g (0,32 mol, 57 mol%) Thioharnstoff wurden in Portionen als Feststoff zugegeben und die Mischung 16 h bei 20 °C gerührt. Ungelöstes Material wurde abfiltriert und Ethanol bei vermindertem Druck abdestilliert bis die Kristallisation des Produktes einsetzte. Es wurde etwas Aceton zugegeben, das Produkt abfiltriert und im Vakuum getrocknet. Ausbeute 31,0 g (148 mmol, 26%) feine weiße Kristalle, HPLC-Reinheit 99,3%.
¹H-NMR (DMSO-d₆): 1,40 (s, 6H), 3,07 (s, 2H), 9,74 (br, 4H).

### Beispiel 8: Synthese von [5,5-Dimethyl(4,5-dihydroisoxazol-3-yl)]thiocarboxamidin Hydrochlorid ausgehend von Acetonoxim

0,37 g (3,5 mmol, 1,0 mol%) N-Methylanilin wurden in 60 g n-Pentan gelöst. Bei 0 °C wurden zu dieser Lösung 0,40 g (3,5 mmol, 1,0 mol%) Trifluoressigsäure gegeben und die resultierende Suspension 10 min gerührt. 25,0 g (0,342 mol, 100 mol%) Acetonoxim wurden zugegeben und die Mischung auf 24-26 °C erwärmt. Anschließend wurden 29,4 g (0,350 mol, 102 mol%) 3-Methyl-2-butenal innerhalb von 2 h zugetropft während die Temperatur bei 25 °C gehalten wurde. Es wurde 60 h bei 20 °C gerührt und die Reaktion durch Zugabe von 1,5 g (18 mmol, 5 mol%) 3-Methyl-2-butenal in 16 h vervollständigt. Das gebildete Aceton wurde unter vermindertem Druck entfernt, 80 g tert-Butanol zugegeben und davon 10 g durch Destillation über eine 20 cm Vigreux-Kolonne entfernt. 26,0 g (0,366 mol, 107 mol%) Chlor wurden getaucht bei 15-20 °C innerhalb von 2 h eingegast und die Reaktionsmischung anschließend 15 min mit Stickstoff gespült. Es wurden 23,5 g (0,31 mol, 90 mol%) Thioharnstoff in Portionen bei 20 °C zugegeben bis sich laut HPLC ein vollständiger Umsatz des gebildeten 3-Chlor-4,5-dihydro-5,5-dimethylisoxazolins zeigte. Das Lösungsmittel wurde unter vermindertem Druck entfernt, der Rückstand in Petrolether suspendiert und die überstehende Lösung verworfen. Das Rohprodukt wurde in Ethanol:Chlorbenzol / 2,5:1 bei 80 °C gelöst, unlösliche Anteile abfiltriert, das Ethanol unter vermindertem Druck entfernt und die Kristallisation durch Abkühlen und Zugabe von Aceton initiiert. Das Produkt wurde abfiltriert, mit Aceton gewaschen und im Vakuum getrocknet. Es wurden 39,2 g (0,187 mol, Ausbeute 55%) [5,5-Dimethyl(4,5-dihydroisoxazol-3-yl)]thio-carboxamidin Hydrochlorid als farblose Kristalle erhalten.
Schmelzpunkt 146-147°C.

### Beispiel 9: Synthese von 3-[(5-Difluormethoxy-1-methyl-3-trifluormethylpyrazol-4-yl)-methylsulfonyl]-4,5-dihydro-5,5-dimethylisoxazol ausgehend von [5,5-Dimethyl-(4,5-dihydroisoxazol-3-yl)]thiocarboxamidin Hydrochlorid

### A) 3-[(5-Hydroxy-1-methyl-3-trifluormethylpyrazol-4-yl)-methylthio]-4,5-dihydro-5,5-dimethylisoxazol

1,49 g (97%ig, 36 mmol, 300 mol%) Natriumhydroxid wurden in 12 g Wasser gelöst und portionsweise wurden 2,0 g (12 mmol, 100 mol%) 5-Hydroxy-1-methyl-3-trifluormethylpyrazol zugegeben. Zu der klaren Lösung wurde bei 24 °C innerhalb von 65 min Formaldehydlösung (36,5 % in Wasser, 2,97 g, 36 mmol, 300 mol%) zugetropft und 90 min bei dieser Temperatur gerührt. Anschließend wurden 3,12 g (92%ig, 14 mmol, 120 mol%) [5,5-Dimethyl-(4,5-dihydroisoxazol-3-yl)]thiocarboxamidin Hydrochlorid, gelöst in 12,8 g Wasser, zugetropft. Es wurde 16 h bei Raumtemperatur gerührt. Man gab 5,9 g Salzsäure (37%ig, 60 mmol, 500 mol%) bei 14-18 °C zu, gefolgt von 12 ml Wasser. Es wurde 16 h bei Raumtemperatur gerührt. Der entstandene Niederschlag wurde filtriert und zweimal mit je 10 ml Wasser und zweimal mit je 15 g n-Hexan gewaschen. Nach Trocknung erhielt man 3,08 g eines kristallinen Rückstandes, der ohne Reinigung weiter eingesetzt wurde.

### B) 3-[(5-Difluormethoxy-1-methyl-3-trifluormethylpyrazol-4-yl)-methylthio]-4,5-dihydro-5,5-dimethylisoxazol

Der erhaltene Rückstand (3,05 g, 10 mmol, 100 mol%) wurde in 30 ml Acetonitril gelöst, 1,22 g (97%ig, 30 mmol, 250 mol%) Natriumhydroxid bei 20-24 °C zugegeben und die Lösung wurde 100 min bei 23 °C gerührt. Es wurde auf 5 °C abgekühlt und 5,34 g (62 mmol, 620 mol%) Chlordifluormethan wurden innerhalb von 45 min bei 5-15 °C eingegast. Es wurde 60 h bei Raumtemperatur gerührt, die Reaktionsmischung mit 15 ml Toluol versetzt und 15 ml Wasser zugegeben. Es wurde 1 ml Salzsäure (37%ig) zugegeben um unlösliche Bestandteile in Lösung zu bringen. Die organische Phase wurde abgetrennt, die wässrige Phase nochmals mit 15 ml Toluol extrahiert und die vereinigten organischen Phasen wurden mit 15 ml Wasser und 15 ml gesättigter Natriumchloridlösung gewaschen. Es wurden 2,9 g bräunliches Öl erhalten, das ohne weitere Reinigung weiter eingesetzt wurde.

### C) 3-[(5-Difluormethoxy-1-methyl-3-trifluormethylpyrazol-4-yl)-methylsulfonyl]-4,5-dihydro-5,5-dimethylisoxazol

2,8 g (7,8 mol) des erhaltenen Öls wurden in 8 ml Essigsäure gelöst und mit 80 mg (0,23 mmol, 3 mol%) Natriumwolframat-Dihydrat versetzt. Es wurde bei 23-34 °C innerhalb von 20 min Wasserstoffperoxid (30%ig, 2,21 g, 20 mmol, 250 mol%) zugetropft und 16 h bei Raumtemperatur gerührt. Das Produkt wurde durch Zugabe von 4 g Wasser und Abkühlen auf 1 °C ausgefällt. Nach einer Stunde bei 10 °C wurde der Feststoff abfiltriert und zweimal mit je 20 g Wasser und 20 ml Petrolether gewaschen. Man erhielt 1,0 g (2,6 mmol) eines Feststoffs.
¹H-NMR (CDCl₃): 6,82 (t, 1 H), 4,60 (s, 2H), 3,87 (s, 3H), 3,10 (s, 2H), 1,51 (s, 6H).

### Vergleichsbeispiel 1: Umsetzung von 5,5-Dimethyl-4,5-Dihydroisoxazol mit Chlorgas in Ethanol

10,0 g (90,2%ig, 91,0 mmol, 100 mol%) 5,5-Dimethyl-4,5-Dihydroisoxazol wurden in 20 g Ethanol gelöst. Bei 15-35 °C wurden 12,0 g (169 mmol, 190 mol%) Chlor getaucht eingegast und die Lösung anschließend 1 h mit Stickstoff gespült. Laut qualitativem HPLC/MS wurde ein Gemisch aus 3-Ethoxy-5,5-dimethyl-4,5-Dihydroisoxazol und 3-Chlor-5,5-dimethyl-4,5-Dihydroisoxazol im Verhältnis 3,5 : 1 erhalten. Die Reaktionsmischung wurde mit 5,6 g (73,6 mmol, 81 mol%) Thioharnstoff versetzt und 16 h bei 22 °C gerührt. Das 3-Ethoxy-5,5-dimethyl-4,5-Dihydroisoxazol hatte sich bei dieser Temperatur nicht umgesetzt und lag neben großen Mengen Thioharnstoff unverändert vor. Die Temperatur wurde für 7 h auf 50 °C erhöht, aber auch anschließend wurden nur Spuren des gewünschten [5,5-Dimethyl(4,5-dihydroisoxazol-3-yl)]thiocarboxamidin Hydrochlorids neben Zersetzungsprodukten per HPLC detektiert.

### Vergleichsbeispiel 2: Säureeinfluss bei Schritt ii), der Umsetzung mit Thioharnstoff

0,5 g (91,4%ig, 3,4 mmol) frisch destilliertes 3-Chlor-4,5-dihydro-5,5-dimethylisoxazol wurde in 5 g tert-Butanol gelöst und bei 25 °C mit 0,25 g (3,3 mmol) Thioharnstoff und 3 Tropfen 32%iger Salzsäure versetzt. Eine Reaktionskontrolle per HPLC ergab nach 3,5 h einen Umsatz zum [5,5-Dimethyl(4,5-dihydroisoxazol-3-yl)]thiocarboxamidin Hydrochlorid von 39%. In einer identisch behandelten Vergleichsprobe ohne Salzsäurezugabe konnte nach 3,5 h kein Umsatz zum Produkt festgestellt werden.

## Patentansprüche

1. Verfahren zur Herstellung 5,5-disubstituierter 4,5-Dihydroisoxazol-3-thiocarboxamidinsalze der Formel (I), wobei
R¹, R² unabhängig voneinander jeweils C₁-C₆-Alkyl oder C₁-C₄-Halogenalkyl;
R³, R⁴ unabhängig voneinander jeweils Wasserstoff, Halogen, C₁-C₆-Alkyl oder C₁-C₄-Halogenalkyl bedeuten; oder
R¹ und R², R³ und R⁴ oder R¹ und R³ zusammen eine C₂-C₅-Alkandiyl-Kette bilden, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann; und
A Chlor oder Brom bedeutet; 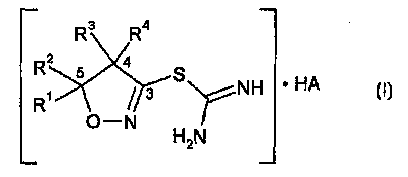
**dadurch gekennzeichnet, dass**
i) 3-unsubstituierte 4,5-Dihydroisoxazole der Formel (II) zunächst mit einem Chlorierungs- oder Bromierungsreagenz zu 3-halogenierten 4,5-Dihydroisoxazolen der Formel (III) umgesetzt werden und 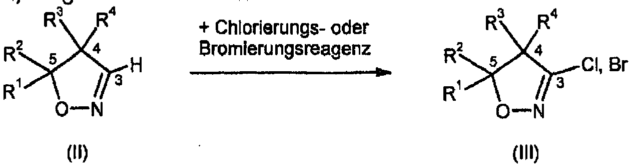
ii) die 3-halogenierten 4,5-Dihydroisoxazole der Formel (III) anschließend mit Thioharnstoff zu den Verbindungen der Formel (I) reagieren, 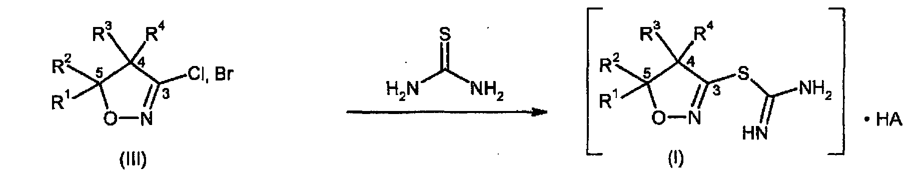
wobei die Schritte i) und ii) im gleichen Lösungsmittel, tert-Butanol oder tert-Amylalkohol, durchgeführt werden.

2. Verfahren nach Anspruch 1, wobei
R¹, R² unabhängig voneinander jeweils C₁-C₆-Alkyl oder C₁-C₄-Halogenalkyl bedeuten; oder
R¹ und R² zusammen eine C₂-C₅-Alkandiyl-Kette bilden, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann; und
R³, R⁴ Wasserstoff bedeuten.

3. Verfahren nach Anspruch 1, wobei
R¹ C₁-C₄-Alkyl;
R² C₁-C₄-Alkyl;
R³ Wasserstoff, Fluor, Chlor, Brom, Methyl, Ethyl; und
R⁴ Wasserstoff, Fluor, Chlor, Brom, Methyl, Ethyl;
bedeuten;

4. Verfahren nach Anspruch 1, wobei
R¹ M ethyl;
R² Methyl;
R³ Wasserstoff; und
R⁴ Wasserstoff; bedeuten.

5. Verfahren nach einem der Ansprüche 1 bis 4, **dadurch gekennzeichnet, dass** das Lösungsmittel tert-Butanol ist.

6. Verfahren nach einem der Ansprüche 1 bis 5, wobei als Chlorierungs- beziehungsweise Bromierungsreagenz elementares Chlor oder Brom verwendet werden.

7. Verfahren nach einem der Ansprüchen 1 bis 6, **dadurch gekennzeichnet dass** das Reaktionsprodukt aus Schritt i), 3-halogenierte 4,5-Dihydroisoxazole der Formel (III), direkt ohne Aufreingung in Schritt ii) mit Thioharnstoff zu 4,5-Dihydroisoxazol-3-thiocarboxamidinsalzen der Formel (I) umgesetzt werden.

8. Verfahren nach Anspruch 7, **dadurch gekennzeichnet, dass** die Schritte i) und ii) in einem Ein-Topf-Verfahren durchgeführt werden

9. Verfahren nach einem der Ansprüche 2 bis 8, **dadurch gekennzeichnet, dass** die 3-unsubstituierten 4,5-Dihydroisoxazole der Formel (II) in einer Vorstufe durch Reaktion eines Oxims der Formel (All) 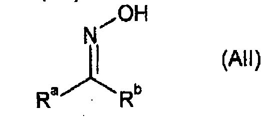 wobei R^{a}, R^{b} unabhängig voneinander jeweils Wasserstoff, C₁-C₆-Alkyl, C₁-C₄-Alkylcarbonyl, Hydroxyimino-C₁-C₄-Alkyl, Phenyl, Phenyl-C₁-C₄-Alkyl oder Phenyl-C₂-C₄-Alkenyl bedeuten, wobei die Phenylringe ein- oder mehrfach durch C₁-C₄-Alkyl, C₁-C₄-Alkoxy, Di-C₁-C₄-Alkylamino, Halogen, Hydroxy oder Nitro substituiert sein können; oder R^{a} und R^{b} zusammen eine C₂-C₅-Alkandiyl-Kette bilden;
mit einer Carbonylverbindung der Formel (AIII), 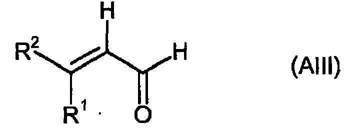 wobei R¹ und R² die in den Ansprüchen 2 bis 4 genannten Bedeutungen haben;
in Anwesenheit eines Säurekatalysators oder eines Säure-Base-Katalysators und gegebenenfalls in Anwesenheit eines organischen Lösungsmittel hergestellt werden.

## Claims

1. A process for preparing 5,5-disubstituted 4,5-dihydroisoxazole-3-thiocarboxamidine salts of the formula (I) where 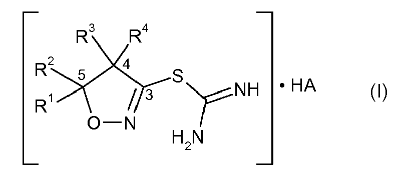
R¹, R² are each independently C₁-C₆-alkyl or C₁-C₄-haloalkyl;
R³, R⁴are each independently hydrogen, halogen, C₁-C₆-alkyl or C₁-C₄-haloalkyl; or
R¹ and R², R³ and R⁴ or R¹ and R³ together form a C₂-C₅-alkanediyl chain which may be mono- to tetrasubstituted by C₁-C₄-alkyl and/or interrupted by oxygen or by optionally C₁-C₄-alkyl-substituted nitrogen; and
A is chlorine or bromine;
wherein
i) 3-unsubstituted 4,5-dihydroisoxazoles of the formula (II) are first reacted with a chlorinating or brominating reagent to give 3-halogenated 4,5-dihydroisoxazoles of the formula (III) and 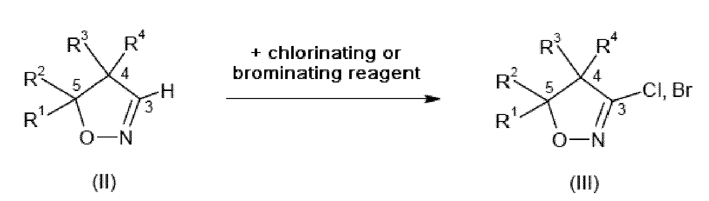
ii) the 3-halogenated 4,5-dihydroisoxazoles of the formula (III) then react with thiourea to give the compounds of the formula (I), 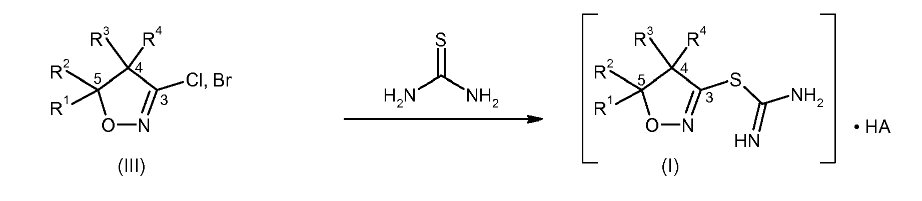
where steps i) and ii) are performed in the same solvent: tert-butanol or tert-amyl alcohol.

2. The process according to claim 1, where
R¹, R² are each independently C₁-C₆-alkyl or C₁-C₄-haloalkyl; or
R¹ and R² together form a C₂-C₅-alkanediyl chain which may be mono- to tetrasubstituted by C₁-C₄-alkyl and/or may be interrupted by oxygen or by optionally C₁-C₄-alkyl-substituted nitrogen; and
R³, R⁴ are each hydrogen.

3. The process according to claim 1, where
R¹ is C₁-C₄-alkyl;
R² is C₁-C₄-alkyl;
R³ is hydrogen, fluorine, chlorine, bromine, methyl, ethyl; and
R⁴ is hydrogen, fluorine, chlorine, bromine, methyl, ethyl.

4. The process according to claim 1, where
R¹ is methyl;
R² is methyl;
R³ is hydrogen; and
R⁴ is hydrogen.

5. The process according to any of claims 1 to 4, wherein the solvent is tert-butanol.

6. The process according to any of claims 1 to 5, wherein the chlorinating or brominating reagent used is elemental chlorine or bromine.

7. The process according to any of claims 1 to 6, wherein the reaction product from step i), 3-halogenated 4,5-dihydroisoxazoles of the formula (III), are reacted directly without purification in step ii) with thiourea to give 4,5-dihydroisoxazole-3-thiocarboxamidine salts of the formula (I).

8. The process according to claim 7, wherein steps i) and ii) are performed in a one-pot process.

9. The process according to any of claims 2 to 8, wherein the 3-unsubstituted 4,5-dihydroisoxazoles of the formula (II) are prepared in a preliminary stage by reaction of an oxime of the formula (AII) 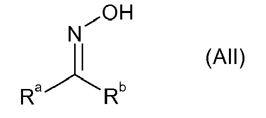 where
R^{a}, R^{b} are each independently hydrogen, C₁-C₆-alkyl, C₁-C₄-alkylcarbonyl, hydroxyimino-C₁-C₄-alkyl, phenyl, phenyl-C₁-C₄-alkyl or phenyl-C₂-C₄-alkenyl, where the phenyl rings may be mono- or polysubstituted by C₁-C₄-alkyl, C₁-C₄-alkoxy, di-C₁-C₄-alkylamino, halogen, hydroxyl or nitro; or R^{a} and R^{b} together form a C₂-C₅-alkanediyl chain;
with a carbonyl compound of the formula (AIII) 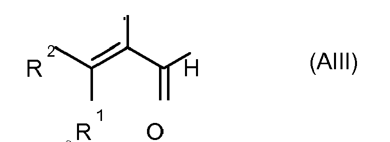 where R¹ and R² are each as defined in claims 2 to 4;
in the presence of an acid catalyst or of an acid-base catalyst and optionally in the presence of an organic solvent.

## Revendications

1. Procédé pour la préparation de sels de 4,5-dihydro-isoxazole-3-thiocarboxamidine disubstitués en 5ème position de formule (I) 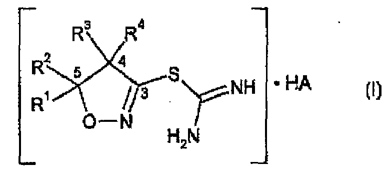
R¹, R² signifiant, indépendamment l'un de l'autre, à chaque fois C₁-C₆-alkyle ou C₁-C₄-halogénoalkyle ;
R³, R⁴ signifiant, indépendamment l'un de l'autre, à chaque fois hydrogène, halogène, C₁-C₆-alkyle ou C₁-C₄-halogénoalkyle ; ou
R¹ et R², R³ et R⁴ ou R¹ et R³ formant ensemble une chaîne C₂-C₅-alcanediyle, qui peut être monosubstituée à tétrasubstituée par C₁-C₄-alkyle et/ou interrompue par oxygène ou par azote, le cas échéant substitué par C₁-C₄-alkyle ; et
A signifiant chlore ou brome ;
**caractérisé en ce que**
i) on transforme d'abord des 4,5-dihydro-isoxazoles non substitués en 3ème position de formule (II) avec un réactif de chloration ou de bromuration en 4,5-dihydro-isoxazoles halogénés en 3ème position de formule (III) et 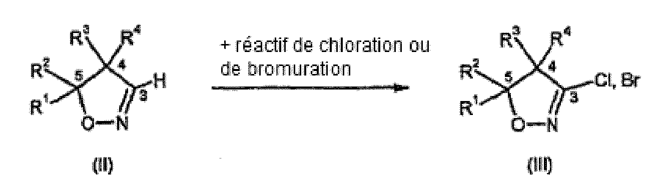
ii) on fait ensuite réagir les 4,5-dihydro-isoxazoles halogénés en 3ème position de formule (III) avec de la thio-urée en composés de formule (I). 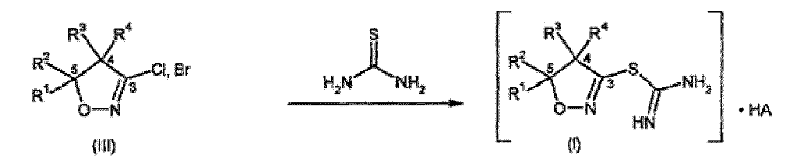
les étapes i) et ii) étant réalisées dans le même solvant, le tert-butanol ou l'alcool tert-amylique.

2. Procédé selon la revendication 1,
R¹, R² signifiant, indépendamment l'un de l'autre, à chaque fois C₁-C₆-alkyle ou C₁-C₄-halogénoalkyle ; ou
R¹ et R² formant ensemble une chaîne C₂-C₅-alcanediyle, qui peut être monosubstituée à tétrasubstituée par C₁-C₄-alkyle et/ou interrompue par oxygène ou par azote, le cas échéant substitué par C₁-C₄-alkyle ; et
R³, R⁴ signifiant hydrogène.

3. Procédé selon la revendication 1,
R¹ signifiant C₁-C₄-alkyle ;
R² signifiant C₁-C₄-alkyle ;
R³ signifiant hydrogène, fluor, chlore, brome, méthyle, éthyle ; et
R⁴ signifiant hydrogène, fluor, chlore, brome, méthyle, éthyle.

4. Procédé selon la revendication 1,
R¹ signifiant méthyle ;
R² signifiant méthyle ;
R³ signifiant hydrogène et
R⁴ signifiant hydrogène.

5. Procédé selon l'une quelconque des revendications 1 à 4, **caractérisé en ce que** le solvant est le tert-butanol.

6. Procédé selon l'une quelconque des revendications 1 à 5, du chlore ou du brome élémentaire étant utilisé comme réactif respectivement de chloration ou de bromuration.

7. Procédé selon l'une quelconque des revendications 1 à 6, **caractérisé en ce que** le produit de réaction de l'étape i), les 4,5-dihydro-isoxazoles halogénés en 3ème position de formule (III), sont transformés directement sans purification dans l'étape ii) avec de la thio-urée en sels 4,5-dihydro-isoxazole-3-thiocarboxamidiniques de formule (I).

8. Procédé selon la revendication 7, **caractérisé en ce que** les étapes i) et ii) sont réalisées dans un procédé dans un pot.

9. Procédé selon l'une quelconque des revendications 2 à 8, **caractérisé en ce que** les 4,5-dihydro-isoxazoles non substitués en 3ème position de formule (II) sont préparés dans une étape préliminaire par réaction d'une oxime de formule (AII) 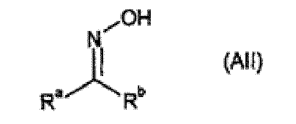
R^{a}, R^{b} signifiant, indépendamment l'un de l'autre, à chaque fois hydrogène, C₁-C₆-alkyle, C₁-C₄-alkylcarbonyle, hydroxylmino-C₁-C₄-alkyle, phényle, phényl-C₁-C₄-alkyle ou phényl-C₂-C₄-alcényle, les cycles phényle pouvant être monosubstitués ou polysubstitués par C₁-C₄-alkyle, C₁-C₄-alcoxy, di-C₁-C₄-alkylamino, halogène, hydroxy ou nitro ; ou R^{a} et R^{b} pouvant former ensemble une chaîne C₂-C₅-alcanediyle ;
avec un composé carbonyle de formule (AIII) 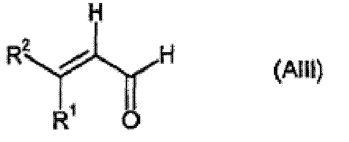
R¹ et R² ayant les significations mentionnées dans les revendications 2 à 4 ;
en présence d'un catalyseur acide ou d'un catalyseur acide-base et le cas échéant en présence d'un solvant organique.
